(19) Europäisches Patentamt
European Patent Office
Office européen des brevets

(11) Veröffentlichungsnummer: **0 141 775**
**B1**

(12) # EUROPÄISCHE PATENTSCHRIFT

(45) Veröffentlichungstag der Patentschrift: **28.10.87**

(51) Int. Cl.⁴: **C 07 C 31/20**, C 07 C 29/03

(21) Anmeldenummer: **84810418.8**

(22) Anmeldetag: **24.08.84**

(54) **Kontinuierliches Verfahren zur Herstellung von 1,2-Alkandiolen.**

(30) Priorität: **30.08.83 US 527923**

(43) Veröffentlichungstag der Anmeldung: **15.05.85 Patentblatt 85/20**

(45) Bekanntmachung des Hinweises auf die Patenterteilung: **28.10.87 Patentblatt 87/44**

(84) Benannte Vertragsstaaten: **AT BE CH DE FR GB IT LI NL SE**

(56) Entgegenhaltungen:
**EP - A - 0 025 940**
**DE - A - 2 937 840**
**DE - B - 2 205 023**
**GB - A - 634 118**
**US - A - 2 492 201**

(73) Patentinhaber: **CIBA-GEIGY AG, Klybeckstrasse 141, CH-4002 Basel (CH)**

(72) Erfinder: **Issler, Heinz, Dr., Weinbergstrasse 20, D-6140 Bensheim (DE)**
Erfinder: **Maul, Rudolf, Dr., Chrodegangstrasse 6a, D-6143 Lorsch/Hessen (DE)**

EP 0 141 775 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein kontinuierliches Verfahren zur Herstellung von 1,2-Alkandiolen der Formel I

$$R-CH(OH)-CH_2-OH \quad (I)$$

in welcher R eine Alkylgruppe mit 3–6 Kohlenstoffatomen bedeutet.

Die 1,2-Alkandiole der Formel I sind wertvolle Zwischenprodukte für die Herstellung von 1-(β-Aryläthyl)-1H-1,2,4-triazolketalen mit antimikrobieller und pflanzenwuchsregulierender Wirkung. Solche 1-(β-Aryläthyl)-1H-1,2,4-triazolketale, ihre Herstellung und Verwendung sind beispielsweise in der US-Patentschrift 4 079 062 beschrieben. Als wichtiger Vertreter dieser Stoffklasse ist das 1-[2-(2,4-Dichlorphenyl)-4-n-propyldioxolan-2-ylmethyl]-1H-1,2,4-triazol zu nennen, das unter der Bezeichnung Propiconazol bekannt ist. Es kann durch Umsetzung von ω-Brom-2,4-dichloracetophenon mit 1,2-Pentandiol zum 2-Brommethyl-2-(2,4-dichlorphenyl)-4n-propyldioxolan und dessen weitere Umsetzung mit 1H-1,2,4-triazol hergestellt werden.

Es ist bekannt, 1-Alkene, wie 1-Octen, 1-Decen, 1-Dodecen, 1-Tetradecen, 1-Hexadecen und 1-Octadecen, durch Umsetzung mit Perameisensäure in Ameisensäure als Lösungsmittel und anschliessende alkalische Verseifung der zunächst gebildeten 1,2-Alkandiolmonoformiate in die entsprechenden 1,2-Alkandiole zu überführen. Dabei wird die benötigte Perameisensäure nicht separat hergestellt, sondern unmittelbar im Reaktionsgemisch erzeugt. Man geht so vor, dass man in eine aus dem 1-Alken und Ameisensäure bestehende Mischung unter Rühren bei etwa 40 °C die notwendige Menge Wasserstoffperoxid einträgt, wobei die Ameisensäure in einer Menge von 10–30 Mol pro Mol 1-Alken eingesetzt und wässeriges Wasserstoffperoxid in einer Konzentration von etwa 25 Gew.-% verwendet wird. Die Reaktionszeit beträgt unter diesen Bedingungen 8–24 Stunden (vgl. J. Amer. Chem. Soc. 68 (1946), 1504–1507). Dieses Verfahren wird gemäss EP-A 25 940 dadurch verbessert, dass man pro Mol 1-Alken 2–6 Mol Ameisensäure einsetzt und Wasserstoffperoxid mit einer Konzentration von 35–98 Gew.-% verwendet und die Umsetzung bei einer Temperatur von 40–80 °C durchführt.

In der DE-A 29 37 840 wird schliesslich ein Verfahren beschrieben, in dem pro Mol Alken weniger als 2 Mol Ameisensäure und weniger als 2 Mol Wasserstoffperoxid verwendet werden, wobei die Konzentration der Ameisensäure 20–100 Gew.-% betragen kann und die Konzentration des verwendeten Wasserstoffperoxids unter 50 Gew.-% liegt. In der GB-A 634 118 wird die Umsetzung von 1-Okten mit 90%iger Ameisensäure und 90%igem Wasserstoffperoxid im Molverhältnis 1:1:1,17 beschrieben.

Die vorgenannten Verfahren werden alle in der Weise durchgeführt, dass man eine Mischung von 1-Alken und Ameisensäure vorlegt und unter intensivem Rühren die notwendige Menge Wasserstoffperoxid einträgt. Diese Arbeitsweise ist für eine grosstechnische Durchführung des Verfahrens insofern nachteilig, als das Ausgangsgemisch wegen der geringen Löslichkeit der als Ausgangsmaterialien verwendeten 1-Alkene in Ameisensäure zu Beginn der Umsetzung in 2 Phasen vorliegt. Die für einen glatten Reaktionsverlauf notwendige vollständige Durchmischung solcher Gemische ist bei der Durchführung des Verfahrens in technischem Massstab sehr schwierig und nur mit grossem Aufwand zu verwirklichen. Ferner ist die bekannte Arbeitsweise für die Umsetzung von niederen 1-Alkenen auch deshalb nachteilig, weil wegen des tiefen Siedepunktes dieser Verbindungen entweder bei sehr tiefen Temperaturen oder unter Druck gearbeitet werden muss. Es ist daher mit den bisher bekannten Verfahren nicht möglich, die 1,2-Alkandiole der Formel I auf einfache und wirtschaftliche Weise im technischen Massstab herzustellen.

Der vorliegenden Erfindung lag die Aufgabe zu Grunde, ein Verfahren zur Herstellung von 1,2-Alkandiolen der Formel I bereitzustellen, das die Nachteile der bekannten Verfahren vermeidet und das die Herstellung der 1,2-Alkandiole der Formel I auf einfache und wirtschaftliche Weise im technischen Massstab ermöglicht.

Gemäss vorliegender Erfindung wird ein kontinuierliches Verfahren zur Herstellung von 1,2-Alkandiolen der Formel I durch Umsetzung eines 1-Alkens der Formel II

$$R-CH=CH_2 \quad (II)$$

in welcher R die unter Formel I angegebene Bedeutung hat, mit Ameisensäure und Wasserstoffperoxid und anschliessende Verseifung des gebildeten Alkandiolmonoformiats vorgeschlagen, das dadurch gekennzeichnet ist, dass man das 1-Alken der Formel II, 70–100%ige Ameisensäure und 50–100%iger Wasserstoffperoxid im Molverhältnis 1,0:0,8–2,0:1,0–1,5 bei einer Temperatur von 0–60° in eine erste Reaktionsstufe eindosiert, das die erste Reaktionsstufe verlassende Reaktionsgemisch in einer zweiten Reaktionsstufe bei 30–60 °C hält, das aus der zweiten Reaktionsstufe austretende Reaktionsgemisch in eine dritte Reaktionsstufe überführt, in der eine Temperatur von 60–80 °C und durch Zugabe einer konzentrierten wässrigen Alkali-Lösung von Alkalimetallhydroxid, -carbonat oder -hydrogencarbonat ein pH-Wert von 10–11 aufrechterhalten wird, und aus dem aus der dritten Reaktionsstufe austretenden Reaktionsgemisch das gebildete 1,2-Alkandiol der Formel I durch Extraktion mit einem organischen Lösungsmittel abtrennt und aus dem Extrakt durch Abdestillieren des Lösungsmittels gewinnt.

Die 1-Alkene der Formel II sind in einer Reinheit von über 90% im Handel erhältlich und können direkt in dieser Form verwendet werden. Geeignete 1-Alkene der Formel II sind beispielsweise 1-Penten, 1-Hexen, 1-Hepten, 1-Octen, 3-Methyl-1-penten, 4-Methyl-1-penten, 3-Methyl-1-hexen, 2,4-Dimethyl-1-penten und 2,5-Dimethyl-1-hexen. Ein bevorzugtes 1-Alken der Formel II ist 1-Penten.

Die Ameisensäure kann in wasserfreier oder wasserhaltiger Form verwendet werden. Vorzugsweise verwendet man Ameisensäure mit einer Konzentration von 80–96%.

Es hat sich bei der Durchführung des erfindungsgemässen Verfahrens als vorteilhaft erwiesen, der Ameisensäure 0,5–6 Gew.-% Schwefelsäure zuzusetzen. Die Schwefelsäure kann in Form von konzentrierter oder in Form von mässig verdünnter Schwefelsäure zugesetzt werden. Vorteilhaft wird etwa 50%ige Schwefelsäure zugesetzt. Die Schwefelsäure kann entweder der Ameisensäure vor der Dosierung in den Reaktor zugemischt oder aber in entsprechender Menge gleichzeitig mit der Ameisensäure in den Reaktor eindosiert werden.

Das Wasserstoffperoxid kann in reiner Form oder als mässig verdünnte wässerige Lösung verwendet werden. Vorzugsweise verwendet man Wasserstoffperoxid mit einer Konzentration von 70–75%.

Innerhalb des bereits angegebenen Bereichs für das Molverhältnis von 1-Alken der Formel II zu Ameisensäure und Wasserstoffperoxid von 1,0:0,8–2,0:1,0–1,5 sind Molverhältnisse von 1-Alken der Formel II zu Ameisensäure und Wasserstoffperoxid von 1,0:1,2–1,5:1,2–1,5 bevorzugt.

Innerhalb der angegebenen Bereiche für die Reaktionstemperatur in den einzelnen Stufen ist in der ersten Stufe eine Reaktionstemperatur von 30–60 °C, in der zweiten Stufe eine Temperatur von 45–60 °C und in der dritten Stufe eine Temperatur von 65–75 °C bevorzugt.

Der Mehrstufen-Reaktor, in dem das erfindungsgemässe Verfahren durchgeführt wird, besteht in der Regel aus mehreren hintereinander geschalteten Einzelreaktoren, die vorteilhaft als Rührkesselkaskade ausgebildet sind. Das erfindungsgemässe Verfahren besteht im wesentlichen aus 3 Stufen, nämlich in einer ersten Stufe, in der die Eindosierung der Reaktanten in das Reaktionsgemisch vorgenommen wird, einer zweiten Stufe in der das nach dem Eindosieren der Reaktanten erhaltene Reaktionsgemisch bis zum vollständigen Ablauf der Reaktion unter definierten Reaktionsbedingungen gehalten wird und in einer dritten Stufe, in der das zunächst gebildete Alkandiolmonoformiat hydrolytisch zum 1,2-Alkandiol der Formel I gespalten wird, bevor das Reaktionsgemisch durch Extraktion und nachfolgende Destillation des Extrakts aufgearbeitet wird. Eine Verfahrensstufe kann aus einem Rührkessel bestehen oder auf mehrere Rührkessel verteilt sein. Dabei wird die Zahl der verwendeten Rührkessel im wesentlichen durch den Durchsatz und die erforderliche Verweilzeit bestimmt. Die Grösse der einzelnen Rührkessel wird in der Regel so bemessen, dass sich aus dem Durchsatz und dem Volumen des einzelnen Kessels rechnerisch eine Verweilzeit von 4–8 Stunden ergibt. Normalerweise sind die einzelnen Einheiten gleich gross. Es können jedoch auch verschieden grosse Einheiten verwendet werden.

Die Dosierung aller Reaktionskomponenten kann im einfachsten Fall im ersten Kessel erfolgen. Es ist jedoch auch möglich und in vielen Fällen vorteilhaft, die Dosierung der Reaktionskomponenten auf zwei oder mehrere Kessel zu verteilen. In diesem Fall wird zweckmässig so gearbeitet, dass man zwar im ersten Kessel bereits die gesamte Menge Ameisensäure und gegebenenfalls Schwefelsäure eindosiert, dass man aber die Zugabe des Alkens der Formel II und des Wasserstoffperoxids auf zwei oder mehrere Kessel verteilt. Dabei wird die insgesamt zuzusetzende Menge an 1-Alken der Formel II und Wasserstoffperoxid in der Regel gleichmässig auf die Anzahl von Kesseln verteilt, in denen man die Dosierung vornimmt.

Die zweite Stufe, in der das Reaktionsgemisch nach vollständiger Eindosierung aller Komponenten ausreagiert, kann ebenfalls aus einem oder mehreren Rührkesseln bestehen. In der zweiten Stufe wird in der Regel eine höhere Reaktionstemperatur gewählt als in der ersten Stufe. Zweckmässig wählt man in der zweiten Stufe die Reaktionstemperatur so, dass aus dem Reaktionsgemisch nicht umgesetztes 1-Alken der Formel I abdestilliert und in die erste Stufe zurückgeführt werden kann.

In der dritten Stufe, die ebenfalls aus einem oder mehreren Rührkesseln bestehen kann, wird dem ausreagierten Reaktionsgemisch das von nicht umgesetztem 1-Alken der Formel I befreit ist, wässriges Alkali in solcher Menge zugesetzt, dass ein pH-Wert von 10–11 resultiert. Dabei dient als wässriges Alkali eine konzentrierte wässrige Lösung eines Alkalimetallhydroxids, beispielsweise Natrium- oder Kaliumhydroxyd, insbesondere 50%ige Natronlauge oder eines Alkalicarbonats oder -hydrogencarbonats, beispielsweise Natriumcarbonat, Kaliumcarbonat, Natriumhydrogencarbonat und Kaliumhydrogencarbonat.

Aus dem Reaktionsgemisch, das aus dem letzten Kessel der Rührkesselkaskade austritt, wird das gebildete 1,2-Alkandiol der Formel I durch Extraktion mit einem geeigneten Lösungsmittel abgetrennt. Als geeignete Lösungsmittel kommen aliphatische und aromatische Kohlenwasserstoffe und Halogenkohlenwasserstoffe, Ester und Ketone in Betracht. Geeignete Lösungsmittel sind beispielsweise Methylenchlorid, Chloroform, Kohlenstofftetrachlorid, 1,2-Dichloräthan, Toluol, Chlorbenzol, Äthylacetat, Butylacetat, Methyläthylketon, Methylisobutylketon und Methyl-tert.butylketon. Besonders geeignete Lösungsmittel sind Methylenchlorid, Chloroform, Äthylacetat, Methylisobutylketon und Methyl-tert.-butylketon. Besonders bevorzugte Lösungsmittel sind Methylisobutylketon und Methyl-tert.butylketon.

Die Extraktion wird vorteilhaft kontinuierlich in

einer Extraktionskolonne durchgeführt, in der das Reaktionsgemisch und das Lösungsmittel im Gegenstrom zueinander geführt werden. Aus dem Extrakt wird das 1,2-Alkandiol der Formel I durch Abdestillieren des Lösungsmittels gewonnen. Diese Destillation kann in einer üblichen Destillationskolonne, beispielsweise einer Füllkörper-, Glocken- oder Siebbodenkolonne durchgeführt werden. Vorteilhaft wird das Lösungsmittel in einem Dünnschicht-Verdampfer entfernt. Das nach Entfernung des Lösungsmittels erhaltene Rohprodukt kann durch Destillation gereinigt werden.

Bei der Inbetriebnahme der Rührkesselkaskade geht man zweckmässig so vor, dass man im ersten Kessel Ameisensäure, der gegebenenfalls eine entsprechende Menge Schwefelsäure zugemischt ist, vorlegt und das Alken und das Wasserstoffperoxid gleichzeitig unter den angegebenen Bedingungen eindosiert. Wenn so viel Alken und Wasserstoffperoxid eindosiert ist, wie es dem Molverhältnis entspricht, bei dem in dem betreffenden Kessel gearbeitet werden soll, so beginnt man zusätzlich mit der Eindosierung von Ameisensäure und gegebenenfalls Schwefelsäure und überführt gleichzeitig eine der Menge der zugeführten Reaktionskomponenten entsprechende Menge Reaktionsgemisch in den nächsten Kessel, wobei diesem Reaktionsgemisch gleichzeitig frisches Alken und Wasserstoffperoxid zugesetzt werden kann.

Die Reaktionskomponenten können entweder einzeln oder als Vormischung in den Reaktor eindosiert werden. Beim Eindosieren der Komponenten als Vormischung werden diese in einer dem Reaktor vorgeschalteten Mischkammer unter hoher Turbulenz gemischt und aus der Mischkammer direkt in den Reaktor eingeführt. Wenn Reaktoren mit vorgeschalteter Mischkammer für die Reaktionskomponenten verwendet werden, so kann im Reaktor selbst auf eine Rührvorrichtung verzichtet werden. Daher können in diesem Fall ausser Rührkesseln auch andere Reaktoren, beispielsweise ein Röhrenreaktor verwendet werden. Es ist dann allerdings zweckmässig, für die einzelnen Verfahrensstufen mindestens je eine Reaktor-Einheit zu verwenden und das Reaktionsgemisch beim Übergang von einer Einheit in die andere jeweils durch eine Mischkammer zu führen, in die gleichzeitig weiteres Ausgangsmaterial eindosiert werden kann.

Mit dem erfindungsgemässen Verfahren wird es möglich, 1,2-Alkandiole der Formel I in einfacher und wirtschaftlicher Weise in ausgezeichneter Ausbeute und Reinheit herzustellen. Ein besonderer Vorteil des erfindungsgemässen Verfahrens besteht darin, dass das Reaktionsgemisch einphasig bleibt. Dies ist weitgehend auch dann der Fall, wenn bei möglichen Über- oder Unterdosierungen Störungen auftreten, da derartige Störungen durch die erfindungsgemässe mehrstufige Arbeitsweise abgepuffert werden. Damit wird das gesamte System bei der erfindungsgemässen Durchführung in einem Mehrstufenreaktor wesentlich belastbarer als in einem Einstufenreaktor.

Ein weiterer wesentlicher Vorteil des erfindungsgemässen Verfahrens ist ferner darin zu sehen, dass auch niedere Alkene der Formel II, wie 1-Penten oder 1-Hexen, ohne Anwendung von Überdruck bei Temperaturen umgesetzt werden können, die deutlich über dem Siedepunkt dieser Substanzen liegen. Beispielsweise kann 1-Penten unter Normaldruck ohne weiteres bei Temperaturen von bis zu 60 °C umgesetzt werden, ohne dass sich die Substanz aus dem Reaktionsgemisch verflüchtigt. Damit ist das erfindungsgemässe Verfahren insbesondere auch zur Umsetzung von niederen Alkenen der Formel II geeignet. Ein weiterer Vorteil des erfindungsgemässen Verfahrens resultiert schliesslich aus der Einbeziehung der Hydrolyse der zunächst gebildeten Alkandiolmonoformiate, da bei dieser Arbeitsweise sowohl auf die destillative Abrennung von Ameisensäure als auch auf die Zerstörung von überschüssigem Wasserstoffperoxid verzichtet werden kann.

Das erfindungsgemässe Verfahren wird durch dasfolgende Beispiel näher erläutert:

Beispiel

Herstellung von 1,2-Pentandiol

Als Reaktor dient eine aus 4 Rührkesseln bestehende Rührkessel-Kaskade. Die einzelnen Kessel R1, R2, R3 und R4 sind mit Rührer, einem Heiz- bzw. Kühlmantel, Dosiervorrichtungen für die Reaktanten bzw. das Reaktionsgemisch aus dem vorangehenden Kessel und einem Bodenauslass versehen. R1 und R2 sind ferner mit einem Rückflusskühler und R3 mit einem Destillieraufsatz zum Abdestillieren von nicht umgesetztem 1-Penten ausgestattet. R1 und R2 haben ein Volumen von je 2 Liter, R3 ein Volumen von 0,5 Liter und R4 ein Volumen von 1 Liter.

Die Ausgangsmaterialien Ameisensäure und Schwefelsäure werden vollständig in R1 eindosiert, während die Dosierung von 1-Penten und Wasserstoffperoxid auf R1 und R2 verteilt wird. Während des Betriebs wird in R1 und R2 eine Temperatur von 40–50 °C, in R3 (Nachreaktor) eine Temperatur von 50–55 °C und in R4 (Verseifungs-Reaktor) eine Temperatur von 70 °C aufrechterhalten. Das aus R3 abdestillierte 1-Penten wird in R1 und R2 recyclisiert und das aus R4 austretende Reaktionsgemisch wird im Gegenstrom zu Äthylacetat durch eine Extraktionskolonne geleitet und der Extrakt in einer Destillationskolonne vom Lösungsmittel befreit und fraktioniert.

| Stoffströme in R1 | | | |
|---|---|---|---|
| 1-Penten | (95%) | 47,0 g/h | 0,64 mol/h |
| Ameisensäure | (85%) | 99,0 g/h | 1,83 mol/h |
| Wasserstoffperoxid | (70%) | 44,0 g/h | 0,91 mol/h |
| Schwefelsäure | (50%) | 3,0 g/h | 0,02 mol/h |

| | | | |
|---|---|---|---|
| Stoffströme in R2 | | | |
| Reaktionsgemisch aus R1 | | | |
| 1-Penten | (95%) | 47,0 g/h | 0,64 mol/h |
| Wasserstoffperoxid | (70%) | 44,0 g/h | 0,91 mol/h |
| Stoffstrom in R3 | | | |
| Reaktionsgemisch aus R2 | | | |
| Stoffströme in R4 | | | |
| Reaktionsgemisch aus R3 | | | |
| Natriumhydroxid | (50%) | ~ 154 g/h | ~ 1,93 mol/h |
| Stoffströme in Extraktion | | | |
| Reaktionsgemisch aus R4 | | | |
| Äthylacetat | | ~ 650 g/h | |
| Stoffstrom in Destillation | | | |
| Extrakt | | ~ 750 g/h | |
| 1,2-Pentandiol: | | 93,0 g/h | 0,89 mol/h |

Ausbeute: 70% d.Theorie bezogen auf 1-Penten.

**Patentansprüche**

1. Kontinuierliches Verfahren zur Herstellung von 1,2-Alkandiolen der Formel I

$$R-CH(OH)-CH_2-OH \quad (I)$$

in welcher R eine Alkylgruppe mit 3–6 Kohlenstoffatomen bedeutet, durch Umsetzung eines 1-Alkens der Formel II

$$R-CH=CH_2 \quad (II)$$

in welcher R die unter Formel I angegebene Bedeutung hat, mit Ameisensäure und Wasserstoffperoxid und anschliessende Verseifung des gebildeten Alkandiolmonoformiats, dadurch gekennzeichnet, dass man das 1-Alken der Formel II, 70–100%ige Ameisensäure und 50–100%iger Wasserstoffperoxid im Molverhältnis 1,0:0,8–2,0:1,0–1,5 bei einer Temperatur von 0–60 °C in eine erste Reaktionsstufe eindosiert, das die erste Reaktionsstufe verlassende Reaktionsgemisch in einer zweiten Reaktionsstufe bei 30–60 °C hält, das aus der zweiten Reaktionsstufe austretende Reaktionsgemisch in eine dritte Reaktionsstufe überführt, in der eine Temperatur von 60–80 °C und durch Zugabe einer konzentrierten wässrigen Lösung von Alkalimetallhydroxid, -carbonat oder -hydrogencarbonat ein pH-Wert von 10–11 aufrechterhalten wird, und aus dem aus der dritten Reaktionsstufe austretenden Reaktionsgemisch das gebildete 1,2-Alkandiol der Formel I durch Extraktion mit einem organischen Lösungsmittel abtrennt und aus dem Extrakt durch Abdestillieren des Lösungsmittels gewinnt.

2. Verfahren nach Anspruch 1, dadurch gekennzeichnet, dass man 80–96%ige Ameisensäure verwendet.

3. Verfahren nach Anspruch 1, dadurch gekennzeichnet, dass man der verwendeten Ameisensäure 0,5–6 Gew.-% Schwefelsäure zusetzt.

4. Verfahren nach Anspruch 1, dadurch gekennzeichnet, dass man 70–75%iges Wasserstoffperoxid verwendet.

5. Verfahren nach Anspruch 1, dadurch gekennzeichnet, dass man das 1-Alken der Formel II, Ameisensäure und Wasserstoffperoxid in einem Molverhältnis von 1,0:1,2–1,5:1,2–1,5 verwendet.

6. Verfahren nach Anspruch 1, dadurch gekennzeichnet, dass man in der ersten Stufe eine Reaktionstemperatur von 30–60 °C, in der zweiten Stufe einer Temperatur von 45–60 °C und in der dritten Stufe eine Temperatur von 65–75 °C anwendet.

7. Verfahren nach Anspruch 1, dadurch gekennzeichnet, dass man das Reaktionsgemisch durch Zugabe einer konzentrierten wässrigen Lösung eines Alkalimetallhydroxids, insbesondere durch Zugabe von 50%iger Natronlauge, auf pH 10–11 stellt.

8. Verfahren nach Anspruch 1, dadurch gekennzeichnet, dass man das gebildete 1,2-Alkandiol der Formel I durch Extraktion mit einem Lösungsmittel aus der Gruppe aliphatische und aromatische Kohlenwasserstoffe und Halogenkohlenwasserstoffe, Ester und Ketone aus dem Reaktionsgemisch abtrennt.

9. Verfahren nach Anspruch 1, dadurch gekennzeichnet, dass man das gebildete 1,2-Alkandiol der Formel I durch Extraktion mit Methylenchlorid, Chloroform, Kohlenstofftetrachlorid, 1,2-Dichloräthan, Toluol, Chlorbenzol, Äthylacetat, Butylacetat, Methyläthylketon, Methylisobutylketon oder Methyl-tert.butylketon aus dem Reaktionsgemisch abtrennt.

10. Verfahren nach Anspruch 1, dadurch gekennzeichnet, dass man das gebildete 1,2-Alkandiol der Formel I durch Extraktion mit Methylenchlorid, Chloroform, Äthylacetat, Methylisobutylketon oder Methyl-tert.butylketon aus dem Reaktionsgemisch abtrennt.

11. Verfahren nach Anspruch 1, dadurch gekennzeichnet, dass man das gebildete 1,2-Alkandiol der Formel I durch Extraktion mit Methylisobutylketon oder Methyl-tert.-butylketon aus dem Reaktionsgemisch abtrennt.

12. Verfahren nach Anspruch 1, dadurch gekennzeichnet, dass man als 1-Alken der Formel II 1-Penten verwendet.

13. Verfahren nach Anspruch 1, dadurch gekennzeichnet, dass man als Reaktor eine Rührkessel-Kaskade verwendet.

**Revendications**

1. Procédé continu pour la préparation des 1,2-alcane-diols de formule I

$$R-\underset{\displaystyle OH}{\underset{|}{C}}H-CH_2-OH \qquad (I)$$

dans laquelle R représente un groupe alkyle en C3–C6, par réaction d'un 1-alcène de formule II

$$R-CH=CH_2 \qquad (II)$$

dans laquelle R a les significations indiquées en référence à la formule I, avec l'acide formique et le peroxyde d'hydrogène suivie d'une saponification du monoformiate d'alcane-diol formé, caractérisé en ce que l'on introduit dans un premier stade de réaction le 1-alcène de formule II, de l'acide formique à une concentration de 70 à 100% et du peroxyde d'hydrogène à une concentration de 50 à 100% dans des proportions molaires relatives de 1,0:0,8 à 2,0:1,0 à 1,5 à une température de 0 à 60 °C, on maintient le mélange de réaction quittant le premier stade de réaction à une température de 30 à 60 °C dans un deuxième stade de réaction, on transfère le mélange de réaction sortant du deuxième stade de réaction dans un troisième stade de réaction dans lequel on maintient une température de 60 à 80 °C et, par addition d'une solution d'hydroxyde, de carbonate ou de bicarbonate de métal alcalin, un pH de 10 à 11, on sépare le 1,2-alcane-diol de formule I formé du mélange de réaction sortant du troisième stade de réaction par extraction à l'aide d'un solvant organique et on l'isole de l'extrait par distillation du solvant.

2. Procédé selon la revendication 1, caractérisé en ce que l'on utilise de l'acide formique à une concentration de 80 à 96%.

3. Procédé selon la revendication 1, caractérisé en ce que l'on ajoute de 0,5 à 6% en poids d'acide sulfurique à l'acide formique utilisé.

4. Procédé selon la revendication 1, caractérisé en ce que l'on utilise du peroxyde d'hydrogène à une concentration de 70 à 75%.

5. Procédé selon la revendication 1, caractérisé en ce que l'on utilise le 1-alcène de formule II, l'acide formique et le peroxyde d'hydrogène dans des proportions molaires relatives de 1,0:1,2 à 1,5:1,2 à 1,5.

6. Procédé selon la revendication 1, caractérisé en ce que l'on observe une température de réaction de 30 à 60 °C au premier stade, une température de 45 à 60 °C au deuxième stade et une température de 65 à 75 °C au troisième stade.

7. Procédé selon la revendication 1, caractérisé en ce que l'on règle le mélange de réaction à un pH de 10 à 11 par addition d'une solution aqueuse concentrée d'un hydroxyde de métal alcalin, en particulier par addition de lessive de soude à 50%.

8. Procédé selon la revendication 1, caractérisé en ce que l'on sépare le 1,2-alcane-diol de formule I du mélange de réaction par extraction à l'aide d'un solvant du groupe des hydrocarbures et hydrocarbures halogénés aliphatiques et aromatiques, des esters et des cétones.

9. Procédé selon la revendication 1, caractérisé en ce que l'on sépare le 1,2-alcane-diol de formule I du mélange de réaction par extraction à l'aide du chlorure de méthylène, du chloroforme, du tétrachlorure de carbone, du 1,2-dichloréthane, du toluène, du chlorobenzène, de l'acétate d'éthyle, de l'acétate de butyle, de la méthyléthylcétone, de la méthylisobutylcétone ou de la méthyl-tert-butylcétone.

10. Procédé selon la revendication 1 caractérisé en ce que l'on sépare le 1,2-alcane-diol de formule I du mélange de réaction par extraction à l'aide du chlorure de méthylène, du chloroforme, de l'acétate d'éthyle, de la méthylisobutylcétone ou de la méthyl-tert-butylcétone.

11. Procédé selon la revendication 1, caractérisé en ce que l'on sépare le 1,2-alcane-diol de formule I du mélange de réaction par extraction à l'aide de la méthylisobutylcétone ou de la méthyl-tert-butylcétone.

12. Procédé selon la revendication 1, caractérisé en ce que le 1-alcène de formule II mis en œuvre est le 1-pentène.

13. Procédé selon la revendication 1, caractérisé en ce que l'appareil de réaction consiste en une cascade de réacteurs équipés de dispositifs d'agitation.

**Claims**

1. A continuous process for producing 1,2-alkanediols of the formula I

$$R-\underset{\displaystyle OH}{\underset{|}{C}}H-CH_2-OH \qquad (I),$$

in which R is an alkyl group having 3–6 carbon atoms, by reacting a 1-alkene of the formule II

$$R-CH=CH_2 \qquad (II),$$

in which R is as defined under the formula I, with formic acid and hydrogen peroxide, and subsequently hydrolysing the alkanediol monoformate formed, characterised in that the 1-alkene of the formula II, 70–100% formic acid and 50–100% hydrogen peroxide are introduced in controlled amounts in a molar ratio of 1,0:0,8–2,0:1,0–1,5, at a temperature of 0–60 °C, into a first reaction stage, the reaction mixture leaving the first reaction stage is held at 30–60 °C in a second reaction

stage, the reaction mixture emerging from the second reaction stage is transferred to a third reaction stage in which there is maintained a temperature of 60–80 °C and, by the addition of a concentrated aqueous solution of alkali metal hydroxide, carbonate or hydrogen carbonate, a pH value of 10–11 and, from the reaction mixture leaving the third reaction stage, the resulting 1,2-alkanediol of the formula I is separated by extraction with an organic solvent and recovered from the extract by removal of the solvent by distillation.

2. A process according to claim 1, characterised in that 80–96% formic acid is used.

3. A process according to claim 1, characterised in that 0,5–6% by weight of sulfuric acid is added to the formic acid used.

4. A process according to claim 1, characterised in that 70–75% hydrogen peroxide is used.

5. A process according to claim 1, characterised in that the 1-alkene of the formula II, formic acid and hydrogen peroxide are used in a molar ratio of 1,0:1,2–1,5:1,2–1,5.

6. A process according to claim 1, characterised in that there is used in the first stage a reaction temperature of 30–60 °C, in the second stage a temperature of 45–60 °C and in the third stage a temperature of 65–75 °C.

7. A process according to claim 1, characterised in that the pH value of the reaction mixture is adjusted to 10–11 by the addition of a concentrated aqueous solution of an alkali metal hydroxide, especially by the addition of 50% sodium hydroxide solution.

8. A process according to claim 1, characterised in that the resulting 1,2-alkanediol of the formula I is separated from the reaction mixture by extraction with a solvent selected from the group comprising aliphatic and aromatic hydrocarbons and halogenated hydrocarbons, esters and ketones.

9. A process according to claim 1, characterised in that the resulting 1,2-alkanediol of the formula I is separated from the reaction mixture by extraction with methylene chloride, chloroform, carbon tetrachloride, 1,2-dichloroethane, toluene, chlorobenzene, ethyl acetate, butyl acetate, methyl ethyl ketone, methyl isobutyl ketone or methyl tert-butyl ketone.

10. A process according to claim 1, characterised in that the resulting 1,2-alkanediol of the formula I is separated from the reaction mixture by extraction with methylene chloride, chloroform, ethyl acetate, methyl isobutyl ketone or methyl tert-butyl ketone.

11. A process according to claim 1, characterised in that the resulting 1,2-alkanediol of the formula I is separated from the reaction mixture by extraction with methyl isobutyl ketone or methyl-tert-butyl ketone.

12. A process according to claim 1, characterised in that 1-pentene is used as 1-alkene of the formula II.

13. A process according to claim 1, characterised in that the reactor used is an agitator vessel cascade.